# EUROPEAN PATENT SPECIFICATION

(11) **EP 0 401 389 B1**
(45) Date of publication and mention of the grant of the patent: **02.03.1994**
(21) Application number: 90901016.7
(22) Date of filing: 28.12.1989
(51) Int. Cl.: A61K 9/20

(54) **STRESS SCATTERING METHOD IN TABLETING**
VERFAHREN ZUM VERTEILEN DES PRESSDRUCKS BEIM TABLETTIEREN
PROCEDE DE DISPERSION DES CONTRAINTES LORS D'UNE OPERATION DE MISE EN TABLETTES

(30) Priority: 28.12.1988 JP 331821/88
(43) Date of publication of application: 12.12.1990
(73) Proprietor: TAISHO PHARMACEUTICAL CO. LTD, Tokyo 171 (JP)
(72) Inventor: YUASA, Hiroshi Higashigaoka Apartment Room 305, Tokyo 151 (JP); KANAYA, Yoshio, Tokyo 165 (JP); OMATA, Kazuki, Tokyo 152 (JP)
(74) Representative: Lamb, John Baxter
(86) International application number: JP8901323
(87) International publication number: WO9007327

(56) References cited:
- FR-A- 806 622
- FR-A- 961 159
- JP-A-53 142 520
- US-A- 2 183 084
- US-A- 2 410 110
- DERWENT FILE SUPPLIER WPIL, 1985, accession no. 85-307860 [49], Derwent Publications Ltd, London, GB; & JP-A-60 215 365 (TOHO YAKUHIN KOGYO)
- JOURNAL OF PHARMACY AND PHARMACOLOGY, vol. 26, suppl., 1974, page 108P; E. SHOTTON et al.: "The use of gelatin hydrolysate to improve the compressibility of paracetamol and phenacetin"

## Description

### [Technical Field]

This invention relates to a composition for tableting containing a stress-dispersing agent for reducing the destuction of microcapsules or the denaturation of drug at the time of tableting, and to a method for dispersion of stress at the time of tableting.

### [Background Technique]

Heretofore, microcapsulated powder of medicine, enzyme or low melting substance or the like has been denatured or destroyed by stress at the time of tableting, and there has taken place tableting trouble such as destruction of the microcapsules or disappearance of the enzyme activity, or the like. It has been known that a large amont of carrier, for example a mixture of lactose, micro-crystalline cellulose and starch is necessary to prevent the destruction of microcapsules at the time of tableting. However, it has made the tablet too large to permit it.

Further, there have been disclosed as techiques to reduce the destruction of microcapsules at the time of tableting a technique which comprises compounding waxy substance in Japanese Laid-Open Patent Publication No. 142520/1978 and a technique of multilayered tablet which comprises formulating under adjusted tableting pressure in Japanese Patent Publication No. 36893/1982. However, the tablet of Japanese Laid-Open Patent Publication No. 142520/1978 has problems in desintegration property, dissolution property of the drug and hardness of the tablet, and the tablet of Japanese Patent Publication No. 36893/1982 has a drawback of complicated formulation.

Tablet formulations containing foamed or powdered gelatin are dissolved in FR-A-806622 and FR-A-968159, respectively.

As a result of vigorous study for solving the deficient points of these conventional techniques, the present inventors have found that stress at the time of tableting can be dispersed by compounding minute spheres of gelatin to microcapsulated medicine powder, and have completed the present invention based on the finding.

Accordingly, the invention provides a method for tableting a medicinal composition comprising:
admixing the medicinal composition with gelatin to reduce the stress within the medicinal composition produced by tableting, said gelatin being in the form of microspheres having a diameter of 0.001 to 2.0 mm; and
compressing said admixture to form the tablet.

The invention also provides a medicinal composition suitable for tableting comprising:
a medicinally active agent;
an excipient; and
gelatin, in the form of microspheres having a diameter of 0.001 to 2 mm.

There have been known as materials having a shape close to that of minute gelatin spheres used in the present invention minute hollow material in Japanese Laid-Open Patent Publication No. 19033/1985, hollow microballoon in Japanese Laid-Open Patent Publication No. 179132/1985 and vertically spherical gelatin grain in Japanese Laid-Open Patent Publication No. 215365/1985. The size of gelatin minute spheres is 0.001 to 2.0 mm, preferably 0.005 to 2.0 mm in diameter. It is preferred that minute gelatin spheres have a water content of 5 to 15%, and it is preferred that they are prepared using gelatin having a molecular weight of 5,000 to 25,000 and a jelly strength (6:66 w/w%) of 50 to 350 (50 to 350 blooms), further preferably a molecular weight of 8,000 to 20,000 and a jelly strength (6.66 w/w%) of 100 g (200 blooms) or more. It is also possible, if necessary, to compound plasticizer to the minute gelatin spheres. Usable plasticizers include glycerin, propylene glycol, sucrose, glucose, starch syrup, honey, etc.

Process of preparation of minute gelatin spheres is described below:
A warmed aqueous 5 to 50% gelatin solution, to which plasticizer was first added if necessary, was poured into a liquid immiscible with the warmed aqueous gelatin solution (any liquid can be used so long as it is immiscible with an aqueous gelatin solution, and include, for example, liquid paraffin, silicone oil, vanasate, coconut oil, sesame oil, wheat germ oil and safflower oil). The mixture was stirred in a stirrer to make the aqueous gelatin solution minute droplets, and then cooled with stirring to coagulate the minute droplets of aqueous gelatin solution. The coagulated droplets were taken out, dried, washed with organic solvent such as hexane, acetone, xylene, methanol, ethanol or IPA to remove the liquid immiscible with the aqueos gelatin solution, and then further dried.

Besides the thus obtained minute spheres, there can be added to the composition for tableting of the present invention additives, for example, excipient such as starch, lactose, microcrystalline cellulose (MCC), sucrose, glucose, urea, calcium carbonate, magnesium metasilicate aluminate or synthesized aluminum silicate; binder such as polyvinyl alcohol (PVA), polyvinylpyrrolidone (PVP), hydroxypropylmethylcellulose (HPMC), hydroxypropylcellulose (HPC), vinyl acetate, crystalline cellulose, carboxymethylcellulose (CMC), agar, sodium alginate, dextrin, gelatin, glucose, gum arabic or tragacanth gum; disintegrating agent such as CMC-calcium, CMC-sodium, silicic anhydride or hydroxypropylstarch (HPS); lubricant such as stearic acid, magnesium stearate, calcium stearate, talc, potassium hydrogen phosphate, carnauba wax, bees wax exposed to sunlight, light silicic anhydride, synthesized aluminum silicate, natural aluminum silicate or dried aluminum hydroxide gel; absorption accelerator; adsorbent; dye; perfume; etc.

Main drug suitable for tablets of the present invention is microorganism, enzyme or low melting substance, or microcapsulant medicine. Namely, such drug is one which is denatured at the time of tableting or destroyed in its structure. For example, there can be mentioned euteric or gradually releasing microcapsulated aspirin, vitamin, proteolytic enzyme, Lactobacillus bifidus, ibuprofen or the like.

Preparation of a composition for tableting of the present invention can be carried out by adding dried and classified minute spheres of gelatin to main drug and additives, and mixing the mixture. Addition amount of foams and/or minute spheres of gelatin is 5 % by weight to 50 % by weight, preferably 10 % by weight to 40 % by weight.

Various tablets can be obtained by tableting such a mixture according to a general tableting method.

### [Best Mode for Carrying Out the Invention)

This invention is more specifically described according to examples and test examples.

### Examples 1 to 9

(1) Purified water (the Japanese Pharmacopoeia) (150 g) was added to 250 g of gelatin, and the mixtue was warmed in a water bath to 60°C to dissolve the gelatin. After dissolution, this aqueous gelatin solution was poured in 500 g of liquid paraffin warmed to 60°C, and the mixture was stirred (about 1,000 rpm) by a stirrer to make the aqueous gelatin solution spherical droplets having a size of 200 µm to 2.5 mm. Then, the droplets of aqueous gelatin solution were coagulated by cooling of the liquid paraffin, and further coagulated by cooling in a refrigerator of about 5°C for 12 hours. After the coagulation by cooling, minute spheres of the gelatinized droplets of aqueous gelatin solution were taken out from the liquid paraffin, and, after washing off the liquid paraffin with hexane, placed in a dehumidifying drier of a temperature of 23°C and a humidity of 40 % or less and dried for 16 hours to obtain a stress-dispersing agent consisting of minute gelatin spheres. The thus obtained minute gelatin spheres had a diameter of about 100 µm to 1.5 mm and a water content of 11 %.
(2) The above minute gelatin spheres were classified into 3 kinds of L, M and S using a sieve.

**Table 1**

| Size of minute gelatin spheres | | |
|---|---|---|
| Size | Classification (mesh) | Diameter (mm) |
| L | 16 - 12 | 1.00 - 1.41 |
| M | 30 - 16 | 0.59 - 1.00 |
| S | 30 or less | 0.59 or less |

Microcapsule-containing tablets were prepared using these classified minute gelatin spheres according to the formulation in Table 2.

**Table 2**

| Formulation of tablet (in 3 tablets) | | | | | | | | | |
|---|---|---|---|---|---|---|---|---|---|
| Formulation | Example | | | | | | | | |
| | 1 | 2 | 3 | 4 | 5 | 6 | 7 | 8 | 9 |
| AMC (mg) | 120 | 120 | 120 | 300 | 300 | 300 | 480 | 480 | 480 |
| HPC (mg) | 240 | 240 | 240 | 240 | 240 | 240 | 240 | 240 | 240 |
| Erosil (mg) | 24 | 24 | 24 | 24 | 24 | 24 | 24 | 24 | 24 |
| MCC (mg) | 336 | 336 | 336 | 336 | 336 | 336 | 336 | 336 | 336 |
| Minute gelatin sphere-L (mg) | 480 | - | - | 300 | - | - | 120 | - | - |
| Minute gelatin sphere-M (mg) | - | 480 | - | - | 300 | - | - | 120 | - |
| Minute gelatin sphere-S (mg) | - | - | 480 | - | - | 300 | - | - | 120 |
| Total | 1200 | 1200 | 1200 | 1200 | 1200 | 1200 | 1200 | 1200 | 1200 |

In the formulation, AMC is aspirin microcapsule (produced by REONEPOULENC Co.), HPS is hydroxypropyl-starch (produced by Freund Industry Co.), Erosil (trade name, produced by Japan Aerosil Co., Aerosil 200), and MCC is microcrystalline cellulose (produced by Asahi Chemical Industry Co., Ltd., Avicel PH 301). These formulation powder compositions were compression-molded by a single charge type tableting machine (cross section 1 cm², flat type) at a tableting pressure of 98000 KPa (1 t) according to the direct powder compression method into tablets weighing 400 mg per tablet.

### Examples 10 to 14

(1)

**Table 3**

| Formulation for plasticizer-compounded minute gelatin sphere | | | | |
|---|---|---|---|---|
| Raw Material | Minute sphere A | Minute sphere B | Minute sphere C | Minute sphere D |
| Gelatin | 25 g | 25 g | 25 g | 25 g |
| Glycerin | 5 g | 10 g | 15 g | 20 g |
| Purified water | 25 g | 25 g | 25 g | 25 g |

Concentrated glycerin and purified water were added to gelatin in the formulation of Table 5, and the mixture was warmed to 60°C in aqueous solution to dissolve the gelatin. After dissolution, this aqueous gelatin solution was poured into 500 g of liquid paraffin warmed to 60°C, and the mixture was stirred (about 1,000 rpm) by a stirrer to make the aqueous gelatin solution spherical droplets having a size of 200 µm to 2.5 mm. The droplets of aqueous gelatin solution were then coagulated by cooling the liquid paraffin, and further coagulated by cooling in a refrigerator of about 5°C for 12 hours. The resulting minute spheres of droplets of gelatinized aqueous gelatin solution was washed for removal of the liquid paraffin, placed in a dehumidifying drier of a temperature of 23°C and a humidity of 40 % or less, and dried for 16 hours to obtain a stress-dispersing agent consisting of minute gelatin spheres. The minute gelatin spheres had a diameter of about 100 µm to 1.5 mm and a water content of 11 % or less.
(2) The above minute gelatin spheres were classified by a sieve into 16 to 100 mesh. Microcapsule-containing composition for tableting were prepared using these classified minute gelatin spheres according to the formulation of Table 4.

**Table 4**

| Formulation of tablet (in 3 tablets) | | | | |
|---|---|---|---|---|
| Formulation | Example | | | |
| | 10 | 11 | 12 | 13 |
| AMC | 300 | 300 | 300 | 300 |
| HPS | 240 | 240 | 240 | 240 |
| Erosil | 24 | 24 | 24 | 24 |
| MCC | 366 | 366 | 366 | 366 |
| Minute sphere A | 300 | 0 | 0 | 0 |
| Minute sphere B | 0 | 300 | 0 | 0 |
| Minute sphere C | 0 | 0 | 300 | 0 |
| Minute sphere D | 0 | 0 | 0 | 300 |
| Total | 1200 | | | |

In the formulation, AMC is aspirin microcapsule (produced by REONEPOULENC Co.), HPS is hydroxypropyl-starch (produced by Freund Industry Co.), Erosil (trade name, produced by Japan Aerosil Co., Ltd., Aerosil 200), and MCC is microcrystalline cellulose (produced by Asahi Chemical Industry Co., Ltd., Avicel PH 301). These formulation powder compositions were compression-molded by a single charge type tableting machine (cross section 1 cm², flat type) according to the direct powder compression method into tablets each weighing 400 mg.

**Table 5**

| Tableting pressure |
|---|
| Tableting pressure (Kpa;kg) |
| 29400 (300) |
| 58800 (600) |
| 98000 1000 |
| 147000 1500 |
| 196000 2000 |

### Test Example 1

### (Specimen)

- Specimens 1 to 9;: Three of each of the tablets of Examples 1 to 9

Control specimens 1 to 3; Three of each of the tablets prepared by compounding MCC in place of minute gelatin spheres in the formulation of Examples 1, 4 and 7 and carrying out the same procedure as in the respective Examples.

Control specimens 4 to 8; Six of each of the tablets prepared by compounding MCC in place of the gelatin foams in the formulations of Examples 10 to 14 and carrying out the same procedure as in respective Examples.

### (Test method)

Each specimen was subjected to the Second Method in the Elution Test Methods in the Japanese Pharmacopoeia (B-424 to 427 in the Explanation of the Japanese Phamacopoeia XI, the paddle method) using purified water of 37°C, and eluted amount was determined as the average value of eluted amount per minute after 3 to 8 minutes.

### (Detection method)

Detection of aspirin was carried out by measuring the absorbance of aspirin at the maximum absorption wavelength of 275 nm by a spectrophotometer (produced by Hitachi, Ltd., U-3200 type).

### (Results)

Results are indicated in Table 8.

**Table 6**

| Amount of eluted aspirin | | |
|---|---|---|
| Specimen | Eluted amount (mg/minute) | Ratio to control specimen (%) |
| 1 | 0.767 | 91.0 |
| 2 | 0.803 | 95.3 |
| 3 | 0.826 | 98.0 |
| Control 1 | 0.843 | 100.0 |
| 4 | 1.808 | 45.6 |
| 5 | 2.297 | 58.0 |
| 6 | 2.454 | 61.9 |
| Control 2 | 3.963 | 100.0 |
| 7 | 3.738 | 92.5 |
| 8 | 3.445 | 95.5 |
| 9 | 3.888 | 96.5 |
| Control 3 | 4.028 | 100.0 |

When aspirin microcapsules are destroyed by the tableting pressure, the elution rate of aspirin increases. The elution rate of any of the specimens is smaller than that of each of the control specimens 1 to 3, which shows stress-dispersing effect of the minute gelatin solution.

### Test Example 2

### (Specimen)

- Specimens 10 to 14;: Three of each of the tablets of Examples 10 to 14

Control specimens 1 to 3; Three of each of the tablets prepared by compounding MCC in place of minute gelatin spheres in the formulation of Example 10 and carrying out the same procedure as in Example 10.

### (Test method)

This test was carried out in the same manner as in Test Example 1.

### (Results)

Results are indicated in Table 7.

**Table 7**

| Amount of eluted aspirin | | | |
|---|---|---|---|
| Specimen | Tableting pressure [Kpa(kg)] | Eluted amount (mg/min.) | Ratio to control specimen (%) |
| 10 | 29400 (300) | 8.23 | 79.1 |
| 11 | | 8.21 | 78.9 |
| 12 | | 7.53 | 72.3 |
| 13 | | 7.53 | 72.3 |
| Control | | 10.41 | 100.0 |
| 10 | 588000 (600) | 8.74 | 87.5 |
| 11 | | 8.55 | 85.6 |
| 12 | | 7.92 | 79.3 |
| 13 | | 8.04 | 80.5 |
| Control | | 9.99 | 100.0 |
| 10 | 98000 (1000) | 9.71 | 66.2 |
| 11 | | 9.63 | 65.7 |
| 12 | | 8.35 | 57.0 |
| 13 | | 9.95 | 67.9 |
| Control | | 14.66 | 100.0 |
| 10 | 147000 (1500) | 10.92 | 81.4 |
| 11 | | 10.80 | 80.5 |
| 12 | | 9.71 | 72.4 |
| 13 | | 9.95 | 74.1 |
| Control | | 13.42 | 100.0 |
| 10 | 196000 (2000) | 12.02 | 82.4 |
| 11 | | 11.44 | 78.4 |
| 12 | | 9.75 | 66.8 |
| 13 | | 9.36 | 64.2 |
| Control | | 14.59 | 100.0 |

### [Industrial Utilizability]

It has become possible by use of stress-dispersing agent of the present invention to tablet microcapsulated, powder of medicine, enzyme or low melting substance or the like, without denaturation or destruction.

## Claims

1. A method for tableting a medicinal composition comprising:
admixing the medicinal composition with gelatin to reduce the stress within the medicinal composition produced by tableting, said gelatin being in the form of microspheres having a diameter of 0.001 to 2.0 mm; and
compressing said admixture to form the tablet.

2. A medicinal composition suitable for tableting comprising:
a medicinally active agent;
an excipient; and
gelatin, in the form of microspheres having a diameter of 0.001 to 2 mm.

3. The composition of claim 2 containing 5 to 50% by weight of said gelatin.

## Patentansprüche

1. Verfahren zum Tablettieren einer medizinischen Zusammensetzung, welches umfaßt:
Mischen der medizinischen Zusammensetzung mit Gelatine, um die Spannung innerhalb der medizinischen Zusammensetzung, die durch das Tablettieren hervorgerrufen wird, zu reduzieren, wobei die genannte Gelatine in Form von Mikrokügelchen mit einem Durchmesser von 0,001 bis 2,0 mm vorliegt; und
Pressen der genannten Mischung zum Formen von Tabletten.

2. Medizinische Zusammensetzung, geeignet zum Tablettieren, welche umfaßt:
ein medizinisch aktives Mittel;
einen Träger und
Gelatine in Form von Mikrokügelchen mit einem
Durchmesser von 0,001 bis 2 mm.

3. Zusammensetzung nach Anspruch 2, die 5 bis 50 Gew.-% an Gelatine umfaßt.

## Revendications

1. Procédé pour la mise sous forme de comprimés d'une composition médicinale comprenant:
le mélange de la composition médicinale avec de la gélatine pour réduire la tension dans la composition médicinale, laquelle tension est produite par la compression, ladite gélatine étant sous la forme de microsphères ayant un diamètre de 0.001 à 2.0 mm et
la compression dudit mélange pour former le comprimé.

2. Composition médicinale adaptée à une compression comprenant:
un agent médicalement actif;
un excipient et
de la gélatine sous la forme de microsphères ayant un diamètre de 0.001 à 2 mm.

3. Composition de la revendication 2 contenant de 5 à 50 % en poids de ladite gélatine.
